# EUROPEAN PATENT SPECIFICATION

(11) **EP 4 344 680 B1**
(45) Date of publication and mention of the grant of the patent: **11.11.2026**
(21) Application number: 23187086.6
(22) Date of filing: 21.07.2023
(51) Int. Cl.: A61F 2/30, A61F 2/42

(54) **IMPLANTS WITH MODULAR STEMS WITH TENSION/COMPRESSION MECHANISMS**
IMPLANTATE MIT MODULAREN SCHÄFTEN MIT SPANNUNGS-/KOMPRESSIONSMECHANISMEN
IMPLANTS À TIGES MODULAIRES AVEC MÉCANISMES DE TENSION/COMPRESSION

(30) Priority: 28.09.2022 US 202263377423 P; 14.07.2023 US 202318352402
(43) Date of publication of application: 03.04.2024
(73) Proprietor: Wright Medical Technology, Inc., Memphis, TN 38117 (US)
(72) Inventor: VISCARDI, David Charles, Glen Rock, 07452 (US); STROHKIRCH, Terrance W., Memphis, 38103 (US); WEST, Jerry W., Arlington, 38002 (US); MOORE, Jesse G., Germantown, 38138 (US)
(74) Representative: Zimmermann & Partner Patentanwälte mbB

(56) References cited:
- EP-A2- 1 623 686
- US-A- 5 916 268
- US-A1- 2020 237 519
- US-B1- 7 153 326

## Description

### FIELD

The present disclosure is related to modular implants. More specifically, the disclosed systems relate to modular stems and implants, including stems and implants that may be used in a total ankle replacement.
Document US 7,534,246 B2 relates to a prosthesis suited for orthopedic implantation that possesses a multi-piece stem component that supports an artificial joint surface that can articulate with another artificial joint surface in various ways, wherein the prosthesis can be assembled in a snap fit and/or interlocking fashion.
Document US 5,916,268 A relates to a kit for an artificial acetabulum.
Document US 7,153,326 B1 relates to a method and apparatus for use of an offset stem connection.
Document US 2020/237519 A1 relates to shoulder implants and methods of use and assembly.
Document EP 1 623 686 A2 relates to a modular orthopaedic implant system.

### BACKGROUND

Tibia stem components help engage implants where limited bone is available for total ankle arthroplasty. Pistoning or loosening of the implant can present as a long-term complication. Thus, improved tibia stem components that can better engage with the tibia and improve immediate implant stability and reduce implant migration over time are desired.

### SUMMARY

Disclosed herein are implants designed to engage cancellous, and possibly cortical, tibia bone to improve immediate implant stability and reduce implant migration over long term. Although the implants are described as being used in connection with a total ankle replacement, it should be understood that the modular stems and connection techniques described herein may be used with other joints, including the knee, shoulder, and hips, for example.

The present invention is defined in claim 1 and concerns a tibia stem prosthesis for ankle replacement including a first component, a second component, and a coupling member.

In some aspects, the second body of the second component may include a protrusion extending from a shoulder. The protrusion may be sized and configured to be at least partially received in the first portion of the first opening defined by the first component.

In some aspects, each of the first portion of the first opening and the protrusion of the second body may include a respective taper. The respective tapers may be configured to engage one another.

In some aspects, the second portion of the first opening may include at least one first thread. The shaft of the coupling member may include at least one second thread. The at least one second thread may be configured to engage the at least one first thread.

In some aspects, the second opening defined by the second component may include a first portion, a second portion, and a third portion. The first portion may include a taper. The second portion may be sized and configured to at least partially receive the head of the coupling member. The third portion may have a diameter that is greater than a diameter of the shaft of the coupling member and smaller than a diameter of the head portion of the coupling member.

In some aspects, the first opening may include a third portion. The second portion of the first opening may be located between the first portion of the first opening and the third portion of the first opening. The first opening may be sized and configured to receive the head portion of the coupling member.

In some aspects, the coupling member may include a tensioning wire comprising the head portion and the shaft portion. A collet may define a third opening and may include a plurality of flexible arms. The third opening may be sized and configured to receive the shaft portion of the tensioning wire.

In some aspects, the second opening defined by the second component may include a first portion and a second portion. The first portion of the second opening may include at least one thread sized and configured to be engaged by a thread disposed on an outer surface of the collet. The second portion may include taper configured to engage the plurality of flexible arms.

In some aspects, the first portion of the second opening may include a taper sized and configured to engage a protrusion of another component.

In some aspects, a third component may have a third body. The third body may include a pair of spaced apart rails and a protrusion extending from an upper surface of the third body. The protrusion may be sized and configured to be received in at least one of the first opening or the second opening.

In some aspects, the third body may define a fourth opening sized and configured to receive the collet therein. The fourth opening defined by the third body may include a first portion and a second portion. The first portion may include at least one thread configured to engage at least one thread disposed on an outer surface of the collet. The second portion may include a taper configured to engage the plurality of flexible arms.

In some aspects, the first portion may include at least one anti-rotation feature extending from and outer surface. The at least one anti-rotation feature may include a plurality of fins extending from the first end of the first body toward the second end of the first body.

In some aspects, the first portion may include surface texturing to promote bone ingrowth/ongrowth. The surface texturing may include at least one groove formed in an outer surface of the first body. The at least one groove may include at least one circumferential groove. The at least one groove may include at least one helical groove.

In some aspects, the surface texturing may include a texture formed by grit blasting.

In some aspects, the first body may include an elongate cylindrical section disposed between the first end of the first body and the second end of the first body.

The invention may be used in a method including inserting a first component of a prosthesis into a resected joint space disposed between a first bone and a second bone. The first component may be advanced into an intramedullary canal formed in the first bone. A second component of the prosthesis may be inserted into the resected joint space. A coupling component may be inserted into a first opening defined by the first component and into a second opening defined by the second component. The coupling component may be rotated to compress the first component and the second component.

The coupling component may include at least one thread that engages at least one thread disposed within the first opening defined by the first component.

The method may include aligning a protrusion of the second component with the first opening defined by the first component such that the protrusion may be received in the opening defined by the first component.

The method may include inserting a third component into the resected joint space. Rotating the coupling component may include rotating the coupling component until at least a portion of a head portion of the coupling component is received in an opening defined by the third component.

The first bone may be a tibia and the second bone may be a talus.

The method may include inserting the coupling component into the resected joint space between the tibia and the talus through an intramedullary canal formed in the talus and a calcaneus.

The method may include inserting the coupling component into the resected joint space between the tibia and the talus.

The method may include inserting a first component of a prosthesis into a resected joint space disposed between a first bone a second bone. The first component of the prosthesis may include a flexible shaft portion of a tensioning wire extending from a trailing end. The first component may be advanced into an intramedullary canal formed in the first bone, and a second component of the prosthesis may be inserted into the resected joint space such that at least a portion of the flexible shaft portion of the tensioning wire is received in an opening defined by the second component. The tensioning wire may be tensioned to compress the first component and the second component.

The method may include inserting the trailing end of the flexible shaft portion into an opening defined by the first component such that the flexible shaft portion extends through the first component and a head portion of the tensioning wire is received within the opening defined by the first component.

The method may include snipping an excess portion of the flexible shaft portion of the tensioning wire.

The method may include inserting a third component of the prosthesis into the resected joint space such that at least a portion of the flexible shaft portion of the tensioning wire is received in an opening defined by the third component. Tensioning the tensioning wire to compress the first component and the second component may include compressing the first component, second component, and third component.

The method may include inserting the trailing end of the flexible shaft portion into an opening defined by a collet and coupling the collet to the third component of the prosthesis.

The method may include inserting a third component of the prosthesis into the resected joint space such that at least a portion of the flexible shaft portion of the tensioning wire is received in an opening defined by the third component and inserting a tibia tray component into the resected joint space such that a least a portion of the flexible shaft portion of the tensioning wire is received in and through an opening defined by a protrusion extending from an upper surface of the tibia tray component. A collet may be coupled to the tibia tray component.

Tensioning the tensioning wire to compress the first component and the second component may include compressing the first component, second component, third component, and the tibia tray component.

Coupling the collet to the tibia tray component may include inserting the collet into the opening defined by the protrusion of the tibia tray component.

Further disclosed herein is a prosthesis that includes a first component, a second component, and a coupling member, wherein the first component includes a first body extending from a first end to a second end, which defines a first opening that extends inwardly toward the first end, wherein the first opening includes a first portion having a first diameter and a second portion having a second diameter, wherein the second component includes a second body extending from a first end to a second end, wherein the second body defines a second opening that extends through the second body, wherein the coupling member is configured to couple the first component and the second component by compressing the first component and the second component, wherein the coupling member includes a shaft portion and a head portion, and wherein the shaft portion is sized and configured to be received in the first opening and the second opening.

### BRIEF DESCRIPTION OF DRAWINGS

The features of the embodiments described herein will be more fully disclosed in the following detailed description, which is to be considered together with the accompanying drawings wherein like numbers refer to like parts. All drawings are schematic and are not intended to show actual dimensions or proportions.
FIG. 1 illustrates one example of a prosthesis stem in accordance with some embodiments;
FIG. 2 illustrates one example of a component of a prosthesis stem in accordance with some embodiments;
FIGS. 3A-3D illustrate examples of surface texturing that may be applied to prosthesis stem components in accordance with some embodiments;
FIGS. 3E and 3F illustrate examples of stem components having differently shaped tapered regions or protrusions, in accordance with some embodiments;
FIG. 4 illustrates one example of a tibia tray component that may be used with a stem prosthesis in accordance with some embodiments;
FIGS. 4A and 4B illustrate examples of tibia tray components having differently shaped tapered regions or protrusions, in accordance with some embodiments;
FIG. 5 is an isometric view of an assembled prosthesis in accordance with some embodiments;
FIG. 6 is a partially exploded view of a stem of the assembled prosthesis illustrated in FIG. 5 in accordance with some embodiments;
FIG. 7 is an exploded view of the stem of the assembled prosthesis illustrated in FIG. 5 in accordance with some embodiments;
FIG. 8 is a top side view of the assembled prosthesis illustrated in FIG. 5 in accordance with some embodiments;
FIG. 9 is a cross-sectional view taken along line A-A in FIG. 8 in accordance with some embodiments;
FIG. 10 is an isometric view of another example of an assembled prosthesis in accordance with some embodiments;
FIG. 11 is a partially exploded view of a stem of the assembled prosthesis illustrated in FIG. 10 in accordance with some embodiments;
FIG. 12 is an exploded view of the stem of the assembled prosthesis illustrated in FIG. 10 in accordance with some embodiments;
FIG. 13 is a top side view of the assembled prosthesis illustrated in FIG. 10 in accordance with some embodiments; and
FIG. 14 is a cross-sectional view taken along line A-A in FIG. 13 in accordance with some embodiments.

### DETAILED DESCRIPTION

This description of the exemplary embodiments is to be read in connection with the accompanying drawings, which are to be considered part of the entire written description. The drawing figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropiate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. The term "operatively connected" is such an attachment, coupling or connection that allows the pertinent structures to operate as intended by virtue of that relationship.

The methods, systems, and structures described for the ankle herein may be adapted to other applications in arthroplasty, including but not limited to the knee, shoulder, hip, elbow, and other joints.

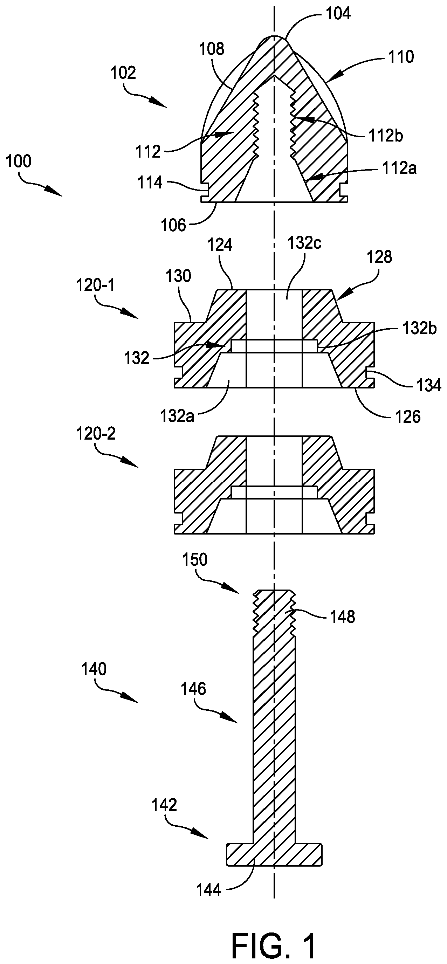

FIG. 1 illustrates one example of a multi-component prosthesis stem in accordance with some embodiments. The stem 100 is component of a larger prosthesis, such as a total ankle replacement system, although the stem 100 may be a standalone prosthesis. The stem shown in FIG. 1 includes a first component 102, one or more second components 120, and a coupling member 140. In the example illustrated in FIG. 1 two components 120-1, 120-2 are provided, although it should be understood that other combinations of components may be provided. For example, fewer (e.g., one) or more components 120 may be provided. Further, depending on the configuration of the first component 102, additional components 102 may be provided in a prosthesis.

The component 102, which may be referred to as a top or leading component, extends in a longitudinal direction from a first end 104 to a second end 106. The leading component 102 may include a curved outer surface 108 that tapers as it extends from the second end 106 toward the first end 104. In some embodiments, one or more fins or counter-torque/anti-rotation features 110 may extend from the outer surface 108. The fins 110 may extend in a longitudinal direction, although the fins 110 may be disposed in other directions (e.g., helical, radial/circumferential, and combinations thereof). In some embodiments, the fins 110 may be interrupted such that each fin may include a plurality of fin segments as will be understood by one of ordinary skill in the art. Further, the counter-torque/anti-rotation features 110 may be deployable in response to the advancement of the coupling member 140, such as the retractable members radially expanding in response to a linear actuator as disclosed in U.S. Provisional Application No. 63/374,248, filed September 1, 2022.

The second end 106 defines an opening 112 that extends inwardly towards first end 104. Opening 112 includes a first portion 112a and a second portion 112b. The first portion 112a may taper from a first diameter to a second diameter in which the second diameter is smaller than the first diameter. The first portion 112a may be tapered to form a Morse taper, although first portion 112a may be otherwise tapered. For example, instead of being conical, the first portion 112a may be pyramidal and have a triangular or square shape such that the first portion 112a may provide a counter-torque or anti-rotation feature. In some embodiments, the second portion 112b may be threaded. An external engagement feature 114 may be disposed adjacent to the second end 106. The engagement element 114 may take a variety of forms, including one or more flats to facilitate engagement by a tool, such as a wrench. Other examples of engagement elements are disclosed in U.S. Patent Application No. 17/650,116, filed February 2, 2022.

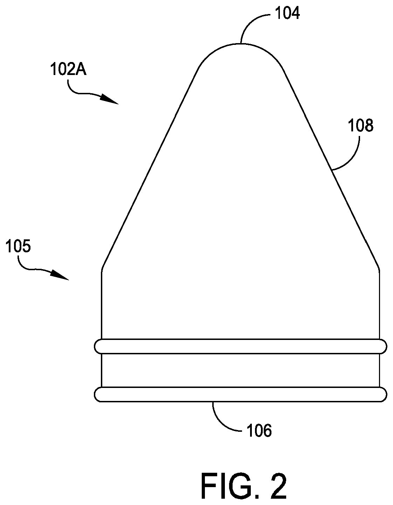

In some embodiments, the external engagement feature 114 may be omitted. Further, in some embodiments, the top component may include a cylindrical portion 105 disposed between the first end 104 and the second end 106, as does the top component 102A shown in FIG. 2. The extended cylindrical portion may increase a length of the top stem component 102A compared to other top stem components, such as top stem component 102 shown in FIG. 2.

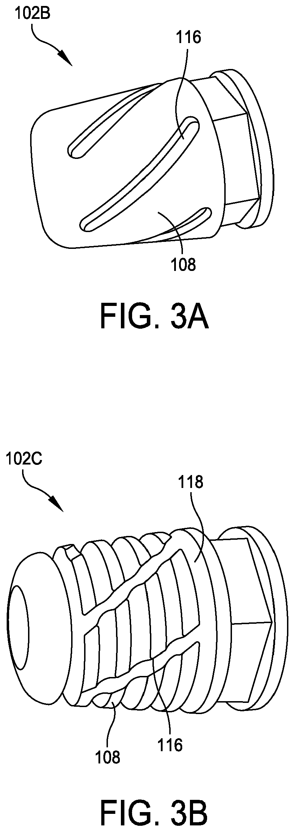

Outer surface 108 may include one or more regions of plasma spray to promote bony ingrowth. However, in some embodiments, the outer surface may not include any plasma spray, but may include other texturing and/or features to promote ingrowth/ongrowth. For example, FIGS. 3A and 3B respectively illustrate examples of top components 102B, 1002C, which may include surface texturing. Referring first to the example illustrated in FIG. 3A, the top component 102B includes a plurality of discrete helical grooves 116 formed in the outer surface 108.

The top component 102C shown in FIG. 3B may include a plurality of helical grooves 116 and a plurality of radial/circumferential grooves 118 that may intersect the helical grooves 116. Forming grooves 116, 118 in the outer surface 108 of the top components 102B, 102C advantageously provides space to allow for ingrowth/ongrowth, which may be easier release compared to the ingrowth/ongrowth experienced by a plasma spray. Although not shown, it should be understood that a top component 102 may be provided with only radial/circumferential grooves and that other shapes and orientations of grooves (e.g., longitudinally oriented) may be provided. The outer surface 108 may also be grit blasted, which may be more economical and/or may provide for an implant that has tighter tolerance compared to a plasma sprayed implant.

Referring again to FIG. 1, component 120 may be a middle and/or trailing component that is configured to be coupled to the top component 102. Component 120 may have a body 122 having a generally cylindrical shape and extend in a longitudinal direction from a first end 124 to a second end 126. In some embodiments, the length of the component 120 (e.g., a distance from the first end 124 to the second end 126) may be greater than a width (e.g., a diameter) of the component 120. However, it should be understood that length of the component 120 may be less than a width. In some embodiments, a kit may be provided in which a plurality of components 120 of differing sizes are provided in the kit.

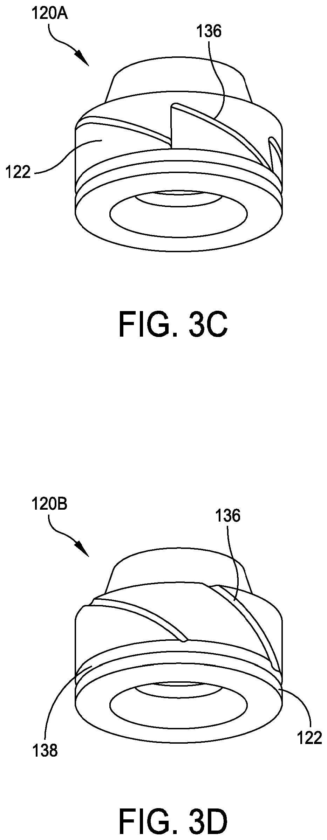

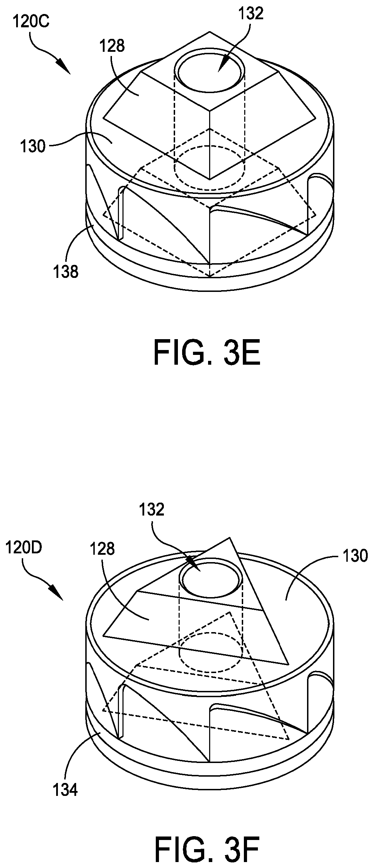

The first end 124 of the component 120 may include a tapered region 128, which may also be referred to as a coupling region, sized and configured to be received in at least a portion of the first portion 112a of the opening 112 of the top component 102. In some embodiments, the tapered region 128 extends from a shoulder 130 of the body 122. The tapered region 128 may be conical (as shown in FIGS. 3C and 3D), pyramidal (as shown in FIGS. 3E and 3F), or may have another shape that facilitates coupling of the component 120 to component 102 as will be understood by one of ordinary skill in the art.

The second end 126 of the component 120 defines an opening 132 that extends inwardly towards first end 124. According to the invention, the opening 132 extends entirely through body 122. Opening 132 may include a first portion 132a, a second portion 132b, and a third portion 132c that are in communication with one another. The first portion 132a may taper from a first diameter or width to a second diameter or width. The second diameter or width may be smaller than the first diameter or width. In some embodiments, the first portion 132a of the opening 132 may be configured to form a Morse tapered connection with another component, such as another component 120 and/or a tibia tray component, such as the tibia tray component 180 shown in FIG. 1. However, the first portion 132a may have other shapes, which may be complementary to the shapes of the protrusions of other components (e.g., a triangular or square pyramid, to list only a few possibilities). The second portion 132b may have a generally cylindrical shape with a third diameter, which may be smaller than the first diameter or width and the second diameter or width. The second portion 132b may be sized and configured to receive a portion of a coupling member, such as the head 144 of coupling member 140. The third portion 132c may also have a generally cylindrical shape and may have a fourth diameter. The fourth diameter may be smaller than the first diameter or width, second diameter or width, and third diameter. In some embodiments, the fourth diameter is sized to provide clearance to receive at least a portion of a coupling member, such as the shaft 146 of the coupling member 140.

An external engagement feature 134 may be disposed adjacent to the second end 126. The external engagement feature 134 may have a similar configuration (e.g., size and shape) to the engagement element 114 of the first component 102 such that the same tool may be used to engagement element 114 and engagement element 134. Accordingly, in some embodiments, the engagement element 134 may include one or more flats to facilitate engagement by a tool, such as a wrench. However, the engagement element may take a variety of forms, including the forms of the engagement elements disclosed in U.S. Patent Application No. 17/650,116. In some embodiments, the component 120 may be provided without an external engagement element 134.

The outer surface of the body 122 of component 120 may include surface texturing to promote ingrowth/ongrowth. For example, FIGS. 3C and 3D illustrate stem components 120A, 120B that includes surface texturing. Referring first to the example illustrated in FIG. 3C, the component 120A includes a plurality of discrete helical grooves 136 formed in the body 122. The component 102B shown in FIG. 3D may include a plurality of helical grooves 136 and a plurality of radial/circumferential grooves 138 that may intersect the helical grooves 136. Forming grooves 136, 138 in the body 122 of the components 120A, 120B advantageously provides space to allow for ingrowth/ongrowth, which may be easier release compared to the ingrowth/ongrowth experienced by a plasma spray. Although not shown, it should be understood that a component 120 may be provided with only radial/circumferential grooves and that other shapes and orientations of grooves (e.g., longitudinally oriented) may be provided. The body 122 may also be grit blasted, which may be more economical and/or may provide for an implant that has tighter tolerance compared to a plasma sprayed implant. In some embodiments, the body 122 may include a plurality of longitudinal fins that extend outwardly from the body 122. In some embodiments, the fins may be arranged in other orientations, such as helical or circumferential.

Coupling member 140 is configured to couple the various components (e.g., the first component 102 and one or more second components 120) of the stem. Coupling member may also be configured to apply a tension to or across one or more of the components 102, 120 and/or be configured as a linear actuator as disclosed in U.S. Provisional Patent Application No. 63/374,248, as will be appreciated by one of ordinary skill in the art. In the example illustrated in FIG. 1, the coupling member 140 includes a head portion 142 and a shaft portion 146. The head portion 142 may have a generally cylindrical shape having a fifth diameter. The fifth diameter may be smaller than the first and second diameters or widths, but may be larger than the third and fourth diameters such that the head portion 142 may be received within the second portion 132b of the opening 132 defined by the second component 120. The head portion 142 may define an engagement feature 144, which may be sized and configured to be engaged by a tool. For example, the engagement feature 144 may include one or more slots or recesses configured to be engaged by a driving tool, such as an Allen wrench/key, a straight screwdriver, a Phillips-head screwdriver), or a Star/Torx driver, to list only a few possibilities.

The shaft portion 146 may extend from the head portion 142 and have a generally cylindrical shape with a sixth diameter. The sixth diameter may be the smallest diameter such that the shaft portion 146 may be received through the one or more second components 120 and at least be partially received within the opening 112. In some embodiments, the shaft portion 146 includes one or more threads 148 disposed at (e.g., adjacent to) the leading end 150. The one or more threads 148 may be configured to engage the threads of the second portion 112b of the first component 102. The one or more threads 148 may include anti-releasing features, such as Loctite, Spiralock, locking washer, or other thread locking features to reduce the chance of thread loosening. Coupling members 140 may be provided in a number of sizes (e.g., lengths and/or diameters) such that stems of various lengths may be formed from multiple stem components. For example, a kit may include a coupling member 140 having a first length that is configured to couple together a first component 102 and a single second component 120. The kit may also include a coupling member 140 having a second length that is configured to couple together a first component 102 and two second components 120. One of ordinary skill in the art will understand that other lengths may be provided. Providing coupling components 140 of differing lengths may provide a surgeon with flexibility to construct a stem of an appropriate length intraoperatively based upon the patient's anatomy.

In use, the patient's anatomy may be prepared, such as by reaming an intramedullary canal in bone, such as is described in U.S. Patent No. 7 534,246. The first component 102 may be inserted into the intramedullary canal. In some embodiments, the first component 102 may be inserted into the intramedullary canal by inserting the first component 102 into a resected joint space formed between a tibia and a talus such that the first component enters the joint space in an anterior-to-posterior direction. The first component 102 may be engaged by a tool and pressed into the intramedullary canal.

A second component (e.g., second component 120-1) may then be coupled to the first component 102 in situ by inserting the second component 120-1 into the resected joint space in an anterior-to-posterior direction. The second component 120-1 may be pressed into engagement with the first component 102 by aligning the two components and inserting the tapered region 128 of the second component 120-1 into the first portion 112a of the opening defined by the first component 102. The construct of the first component 102 and the second component 120-1 may be advanced farther into the intramedullary canal.

A third component, which may be component 120-2, may be coupled to the construct of the first component 102 and second component 120-1 by inserting the third component 120-2 into the resected joint space in an anterior-to-posterior direction. The third component 120-2 may be pressed into engagement with the construct of the first component 102 and the second component 120-1 by inserting the tapered region 128 of the third component 120-2 with the first portion 132a of the opening 132 of the second component 120-1.

When a stem of a desired length has been achieved, the components may be secured to one another through the use of a coupling member 140. For example, the shaft portion 146 of the coupling member 140 may be inserted into the first portion 132a of the opening of the third component 120-2. Depending on the length of the coupling member 140, it may be inserted through the anterior opening or it may be inserted through a path formed in a patient's calcaneus and talus. The coupling member 140 may be advanced axially such that the shaft portion extends through the third component 120-2 and the second component 120-1 (via openings 132) and is received in the opening 112 defined by the first component 102. The threads 148 of the coupling member 148 may engage the threaded portion 112b of the opening 112 defined by the first component 102. The coupling member 140 may be rotated to engage the threads 148 of the coupling member 140 and the first component 102 further. The anti-rotation features 110, which may be embedded within the surrounding bone, may resist rotation of the first component 102 as the coupling member 140 is rotated. In some embodiments, a tool may be used to engage the external engagement feature 134 of the second and/or third components 120-1, 120-2 as the coupling member is rotated to prevent these components from rotating.

The rotation of the coupling member 140 results in linear translation of the coupling member with respect to the first, second, and third components 102, 120-1, 120-2 resulting in the tensioning/compression of the stem components. The tensioning/compression also results in the corresponding tapered surfaces engaging one another and locking the stem components to one another. The stem shown in FIG. 1 may be assembled without the need to thread the individual components together, as required by some conventional multi-component designs. Providing stem components in the absence of threads may reduce the amount of work required to couple together the stem components in situ and may reduce the operating time.

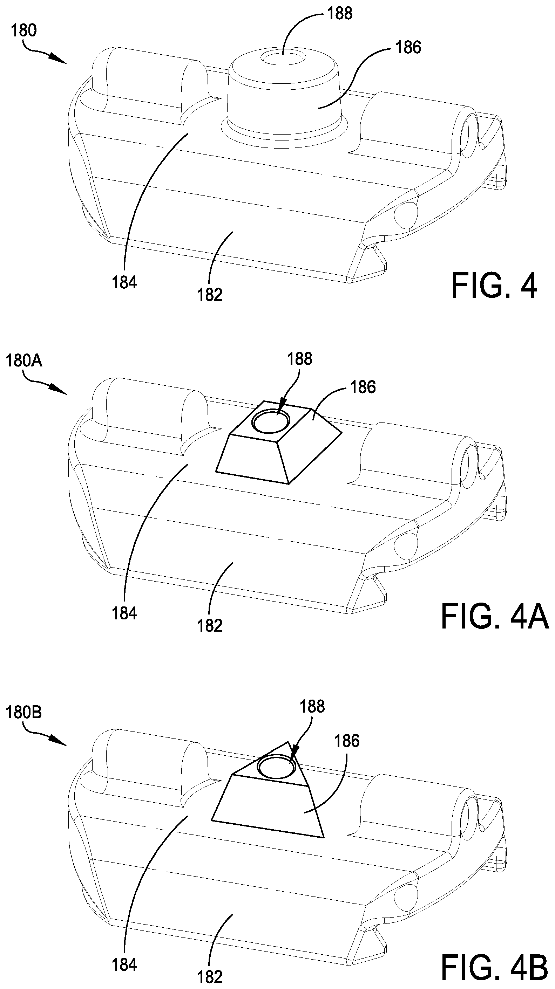

The stem 100 may be coupled to another component of a prosthesis in situ. For example, a tibia tray component, such as the tibia tray component 180 shown in FIG. 4, the tibia tray component 180A shown in FIG. 4A, and the tibia tray component 180B shown in FIG. 4B, may be coupled to the stem 100. For example, a tibia tray component 180, 180A, 180B may be inserted into the resection joint space between the tibia and the talus such that the protrusion 186 extending from an upper surface 184 of the body 182 aligns with the first portion 132a of the opening 132 defined by the third prosthesis component 120-2. As shown in FIGS. 4-4B, the protrusion 186 may be conical (FIG. 4), a square pyramid (FIG. 4A), a triangular pyramid (FIG. 4B), or have other shapes as will be understood by one of ordinary skill in the art. Because the head portion 144 of the coupling member 140 may be received entirely within the second portion 132b of the opening defined by the third prosthesis component 120-2, the all or at least a sufficient portion of the protrusion 186 may be received within the first portion 132a of the opening 132 defined by the third component 120-2. The protrusion 186 may be tapered such that the combination of the protrusion 186 and the first portion 132a of the opening of the third component 120-2 form a tapered connection.

It should be understood that other structures that are suitable for coupling the stem 100 and the tibia tray component 180 may be implemented. For example, the protrusion 186 may define an opening 188 that is sized and configured to receive a coupling member, such as a bolt, screw, or other fastener. The coupling member may be inserted into a corresponding recess defined in the head portion 144 of the coupling member 140 (not shown). One of ordinary skill in the art will understand that other methods of securing the tibia tray component 180 to the stem 100 may be used.

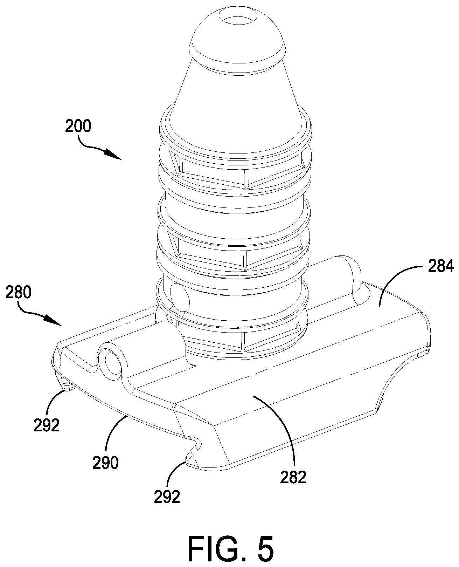

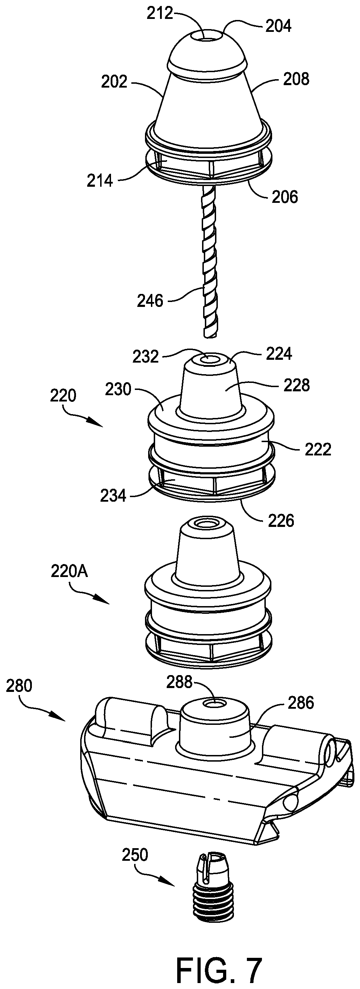

FIGS. 5-9 illustrate an example of a prosthesis in accordance with some embodiments. The prosthesis may include a stem 200 and a tibia tray component 280. The stem 200 may include a first component 202, one or more second components 220, a third component 220A, and a coupling member 240. In the example illustrated in FIGS. 5-9, one component 220 is provided, but it should be understood that other combinations of components may be provided. For example, more components 220 may be provided. Further, depending on the configuration of the first component 202, additional components 202 and/or components 220A may be provided in a prosthesis.

The component 202, which may be referred to as a top or leading component, may extend in a longitudinal direction from a first end 204 to a second end 206. The leading component 202 may include a curved outer surface 208 that tapers as it extends from the second end 206 toward the first end 204. Although not shown, anti-rotation/counter torque features, such as fins, may extend outwardly along the length of the top component 202. Additionally or alternatively, the top component 202 may include surface texturing, such as grooves, plasma spray, grit blasting, and/or other texturing to facilitate bone ingrowth/ongrowth.

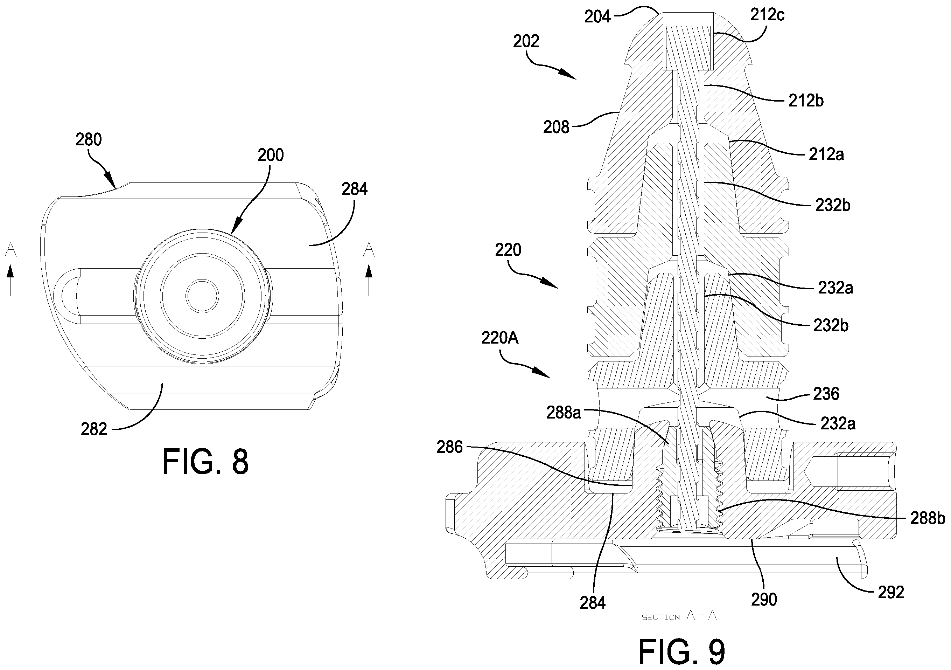

As best seen in FIG. 9, component 202 may define an opening 212 that extends from the first end 204 to the second end 206. Opening 212 may include a first portion 212a, a second portion 212b, and a third portion 212c that are in communication with one another. The first portion 212a may taper from a first diameter located at the second end 206 to a second diameter such that the second diameter smaller than the first diameter. The first portion 212a may be tapered to form a Morse taper, although first portion 212a may be otherwise tapered. The second portion 212b may have a third diameter that is smaller than the first and second diameters. The third portion 212c may extend inwardly from the first end 204 and have a fourth diameter that is greater than the third diameter.

In some embodiments, an external engagement feature 214 may be disposed adjacent to the second end 206. The engagement element 214 may take a variety of forms, including one or more flats to facilitate engagement by a tool, such as a wrench. Other examples of engagement elements are disclosed in U.S. Patent Application No. 17/650,116, filed February 2, 2022, the entirety of which is incorporated herein by reference. In some embodiments, the external engagement feature 214 may be omitted. Further, in some embodiments, the top component may include a cylindrical portion disposed between the first end 204 and the second end 206 such that the top component 202 may have a similar shape to the shape of the top component 102A shown in FIG. 2.

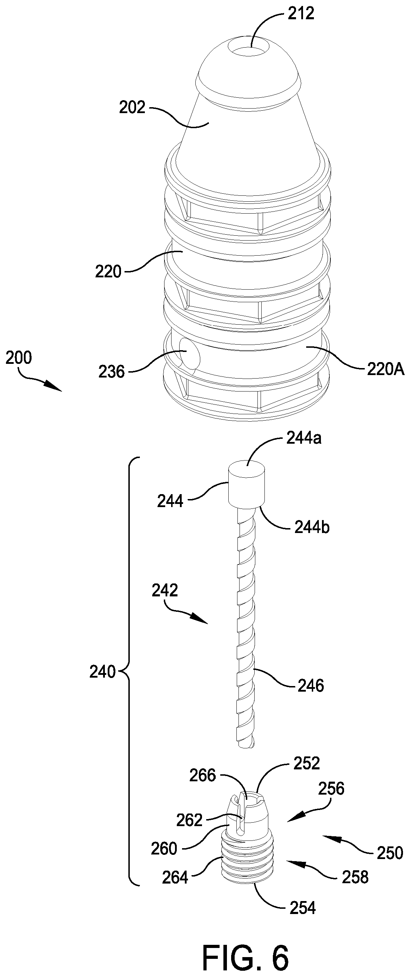

Referring to FIGS. 6 and 9, component 220 may be a middle and/or trailing component that may be configured to be coupled to the top component 202. Component 220 may have a body 222 having a generally cylindrical shape and extend in a longitudinal direction from a first end 224 to a second end 226. In some embodiments, the length of the component 220 (e.g., a distance from the first end 224 to the second end 226) may be greater than a width (e.g., a diameter) of the component 220. However, it should be understood that length of the component 220 may be less than a width. In some embodiments, a kit may be provided in which a plurality of components 220 of differing sizes are provided in the kit.

The first end 224 of the component 220 may include a tapered region 228, which may also be referred to as a coupling region, that may be sized and configured to be received in at least a portion of the first portion 212a of the opening 112 of the top component 202. In some embodiments, the tapered region 228 extends from a shoulder 230 of the body 222. The tapered region 228 may be similar to the tapered region 128 described above. The second end 226 of the component 220 may define an opening 232 that extends inwardly towards first end 224. In some embodiments, the opening 232 may extend entirely through body 222. Opening 232 may include a first portion 232a and a second portion 232b that are in communication with one another. The first portion 232a may taper from a first diameter or width to a second diameter or width. The second diameter or width may be smaller than the first diameter or width. The first portion 232a of the opening 132 may be configured to form a tapered connection (e.g., a Morse taper) with another component, such as another component 220 and/or a tibia tray component, such as the tibia tray component 280 shown in FIGS. 5 and 9. The second portion 232b may have a generally cylindrical shape with a third diameter, which may be smaller than the first diameter or width and the second diameter or width. The second portion 232b may be sized and configured to receive a portion of a coupling member, such as the flexible shaft 246 of the coupling member 240.

An external engagement feature 234 may be disposed adjacent to the second end 226. The external engagement feature 234 may have a similar configuration (e.g., size and shape) to the engagement element 214 of the first component 202 such that the same tool may be used to engagement element 214 and engagement element 234. Accordingly, in some embodiments, the engagement element 234 may include one or more flats to facilitate engagement by a tool, such as a wrench. However, the engagement element may take a variety of forms, including the forms of the engagement elements disclosed in U.S. Patent Application No. 17/650,116. In some embodiments, the component 220 may be provided without an external engagement element 234. The outer surface of the body 222 of component 220 may include surface texturing to promote ingrowth/ongrowth, such as the surface texturing described above with respect to stem components stem components 120A, 120B.

Component 220A may have the same general configuration as component 220 described above, with the addition of a transverse hole 236. Transverse hole 236 may extend entirely through component 220A and be in communication with opening 232. Hole 236 may be sized and configured to receive a tool for disengaging a tapered coupling between component 236 and a protrusion of a tibia tray component as will be understood by one of ordinary skill in the art.

Coupling member 240 may be configured to couple the various components (e.g., the first component 202 and one or more second components 220) of the stem 200 together. In some embodiments, coupling member 240 also may be configured to couple the components of the stem 200 and the stem 200 to another component, such as a tibia tray component 280. Coupling member 240 may also be configured to apply a tension to or across one or more of the components 202, 220 as will be appreciated by one of ordinary skill in the art. In the example illustrated in FIG. 6, the coupling member 240 may include a tensioning wire 242 and a collet 250. Tensioning wire 242 may include a head portion 244 and a flexible shaft portion 246. In some embodiments, the head portion 244 may be a crimped sleeve on the flexible shaft portion 246, which may be a wire, cable, strand, filament, or other elongate flexible member. It should be further understood that the flexible shaft portion 246 may include a single member (e.g., strand or filament) or may include a plurality of strands or filaments, which may be disposed in a braided or unbraided configuration.

The head portion 244 may have a generally cylindrical shape extending from a free end 244a to a coupling end 244b. The head portion 244 may have a diameter that may be smaller than the diameter of the third portion 212c of the opening 212, but larger than a diameter of the second portion 212b such that head portion 244 may be received within the third portion 212c of the opening 212 defined by the top component 202. In some embodiments, the diameter of the head portion 244 is dimensioned such that the head portion 244 may be received in the third portion 212c of the opening 212 in a press-fit or slip-fit manner. Further, although the head portion 244 and the third portion 212c of opening 212 are shown and described as being cylindrical in shape, it should be understood that head portion 244 and third portion 212c of opening 212 may be tapered along their respective lengths or have other shapes.

The flexible shaft portion 246 may be coupled to the coupling end 244b of the head portion 244 and have a diameter that enables the flexible shaft portion to be received within the opening 212 defined by top component 202 and within the opening 232 defined by component 220. The length of the flexible shaft portion 246 may be sufficient so that the flexible shaft portion 246 may be received through a predetermined number of stem components, which may be a maximum possible number of stem components. In some embodiments, the flexible shaft portion 246 may have a length that enables the flexible shaft portion 246 to extend through a maximum possible number of stem components and through a tibia tray component. Providing the flexible shaft 246 with such a length may enable a kit or system to be provided with only a single size tensioning wire 242, which may reduce the number of components in a kit or system. However, it should be understood that multiple tensioning wires 242 may be provided in a kit or system such that a tensioning wire 242 may be provided for each possible size of stem that could be implanted, which may result in a reduction in the amount of material that may need to be removed from the flexible shaft portion 246 during installation.

In some embodiments, the tensioning wire 242 may be provided as a standalone component such that the tensioning wire 242 may be inserted through the top component 202 and any number of components 220 during surgery (or prior to surgery when removed from the kit). However, in some embodiments, the tensioning wire 242 may come preloaded in the top component 242.

Collet 250 may extend from a first end 252, which may be a leading end, to a second end 254, which may be a trailing end. Collet 250 may include a first portion 256 and a second portion 258. The first portion 256 may include a plurality of flexible arms 260 that are separated from one another by a slits 262. In some embodiments, the arms 260 may include a taper along their length to provide a cam surface as will be understood by one of ordinary skill in the art. The second portion 258 may include one or more threads 264. Collet 250 may also define a hole 266 that extends from the first end 252 to the second end 254. Hole 266 may be sized and configured to receive the flexible shaft portion 246 of the tensioning wire 242 and so the flexible arms 260 may lock onto the flexible shaft portion 246 when the flexible arms 260 are compressed.

Collet 250 may be sized and configured to be received within an opening 288 defined by tibia tray component 280. For example, the body 282 of tibia tray component 280 may include an upper surface 284 from which a protrusion 286 extends. The protrusion 286 may define the opening 288 that extends through the body 282 to an internal surface 290, which may be disposed between a pair of spaced apart rails 292, as best seen in FIGS. 5 and 9. Opening 288 may include a first portion 288a and a second portion 288b. First portion 288a may taper along its length and be configured to engage the cam surface of the flexible arms 260 of the collet 250 to facilitate locking of the collet 250 to the flexible shaft portion 246 of the tensioning wire 242. The second portion 288b of the opening 288 may include one or more threads configured to engage the one or more threads 264 of collet 250.

In use, the patient's anatomy may be prepared, such as by reaming an intramedullary canal in bone, such as is described in U.S. Patent No. 7 534,246. The tensioning wire 242 may be preloaded into stem component 202. If tensioning wire 242 is not preloaded into stem component 202, then the flexible shaft portion 246 may be inserted into the opening 212 such that the flexible shaft portion 246 is received first in the third portion 212c, then in the second portion 212b and the first portion 212c. With the tensioning wire 242 coupled to the first component 202, the first component 202 may be inserted into the intramedullary canal. In some embodiments, the first component 202 may be inserted into the intramedullary canal by inserting the first component 202 into a resected joint space formed between a tibia and a talus such that the first component enters the joint space in an anterior-to-posterior direction. The first component 202 may be engaged by a tool and pressed into the intramedullary canal.

A second component, such as component 220, may be coupled to the first component 202 in situ by inserting the component 220 into the resected joint space in an anterior-to-posterior direction. The flexible shaft portion 246 that extends from the first component 202 may be inserted into the opening 232 defined by the component 220. The component 220 may be pressed into engagement with the first component 202 by aligning the two components and inserting the tapered region 228 of the component 220 into the first portion 212a of the opening defined by the first component 202. The construct of the first component 202 and the second component 220 may be advanced farther into the intramedullary canal.

A third component, which may be component 220A, may be coupled to the construct of the first component 202 and second component 220 in situ. For example, the third component 220A may be inserted into the resected joint space in an anterior-to-posterior direction. The flexible shaft portion 246 that extends from the second end 226 of the component 220 may be inserted into the opening 232 defined by the component 220A such that at least a portion of the flexible shaft portion 246 extends from the second end 226 of component 220A. Component 220A may be pressed into engagement with the second component 220 by aligning the two components and inserting the tapered region 228 of component 220A into the first portion 232a of the opening 232 defined by component 220.

When a stem of a desired length has been achieved, the tibia tray component 280 may be coupled to the stem 200. For example, the tibia tray component 280 may be inserted into the resected joint space such that the protrusion 286 may be aligned with the opening 232 defined by the component 220A. The flexible shaft portion 246 may extend from the second end 226 of component 220A and be received in the opening 288 defined by the protrusion 286. The flexible shaft portion 246 also is inserted into the hole 266 defined by the collet 250, which may be preloaded into the tibia tray component 280 or separately coupled to the tibia tray component. A pair of pliers, vice grips, or other tool may be used to pull on the flexible shaft portion 246 of the tensioning wire 242 to compress the components of the stem 200 and the stem 200 onto the tibia tray component 280. The collet 250 may be rotated such that the threads 264 of the collet 250 engage the threaded portion 288b of the opening 288 defined by the tibia tray component 280. The advancement of the collet 250 into the opening 288 results in the flexible arms 260 of the collet being compressed and clamping onto the flexible shaft portion 246 to lock the construct together. Any excess portion of the flexible shaft portion 246 may be cut using a wire cutter, snips, or any other suitable tool.

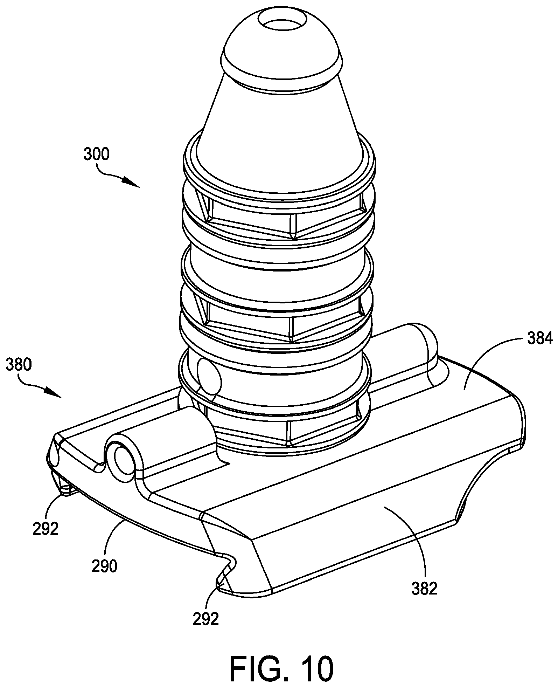

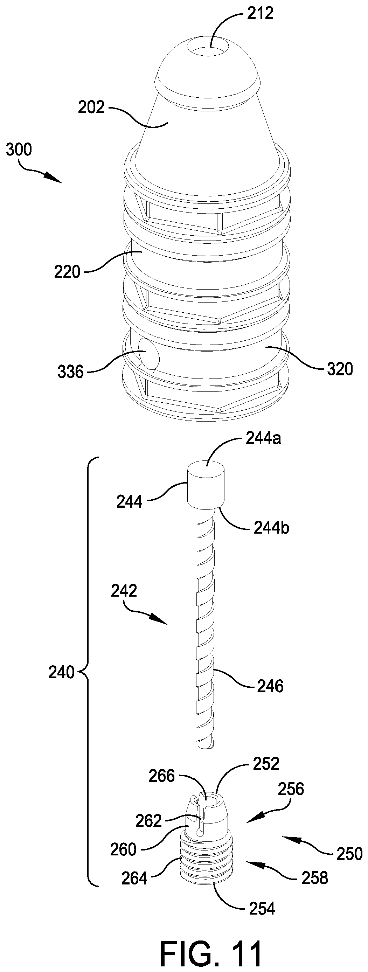

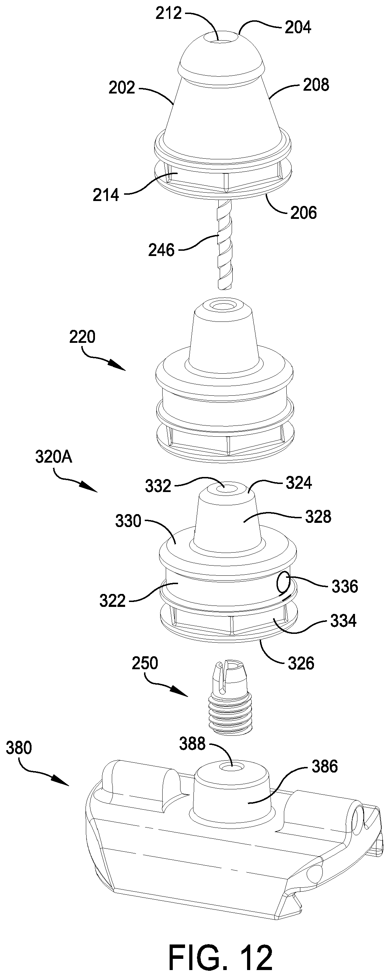

FIGS. 10-14 illustrate another example of a prosthesis that may include a stem and a tibia tray component. Stem 300 may be configured so that each of the stem components may be locked together by a coupling member 240 and be separately coupled to a tibia tray component 380. Stem 300 may include a first component 202 and a second component 220, as described above, and a third component 320. As best seen in FIGS. 11-12, stem component 320 may have a body 322 having a generally cylindrical shape and extend in a longitudinal direction from a first end 324 to a second end 326. In some embodiments, the length of the component 320 (e.g., a distance from the first end 324 to the second end 326) may be greater than a width (e.g., a diameter) of the component 320. However, it should be understood that length of the component 320 may be less than a width. In some embodiments, a kit may be provided in which a plurality of components 320 of differing sizes are provided in the kit.

The first end 324 of the component 320 may include a tapered region 328, which may also be referred to as a coupling region, that may be sized and configured to be received in at least a portion of the first portion 212a of the opening 212 of the top component 202 or in the first portion 232a of the opening 232 of component 220. In some embodiments, the tapered region 328 extends from a shoulder 330 of the body 322. The tapered region may have a conical shape, pyramidal shape, or other shaped in the same or similar manner to the tapered region 128 described above. The second end 326 of the component 320 may define an opening 332 that extends inwardly towards first end 324. In some embodiments, the opening 332 may extend entirely through body 322. Opening 332 may include a first portion 332a, a second portion 332b, and a third portion 332c that are in communication with one another. The first portion 332a may be tapered to provide a Morse tapered connection with another component, such as another component of the stem or with a tibia stem component, including tibia stem component 380. The second portion 332b may be a threaded portion and include one or more threads sized and configured to engage the threads 264 of collet 250. The third portion 332c may have a generally cylindrical shape that tapers along its length such that the third portion 332c may be configured to engage the flexible arms 260 of collet 250 to facilitate locking of the collet 250 to the flexible shaft portion 246 of the tensioning wire 242.

An external engagement feature 334 may be disposed adjacent to the second end 326. The external engagement feature 334 may have a similar configuration (e.g., size and shape) to the engagement element 214 of the first component 202 and engagement element 234 of component 220 such that the same tool may be used to engage engagement elements 214, 234, 334. Accordingly, in some embodiments, the engagement element 334 may include one or more flats to facilitate engagement by a tool, such as a wrench. However, the engagement element may take a variety of forms, including the forms of the engagement elements disclosed in U.S. Patent Application No. 17/650,116. In some embodiments, the component 320 may be provided without an external engagement element 234. The outer surface of the body 322 of component 320 may include surface texturing to promote ingrowth/ongrowth, such as the surface texturing described above with respect to stem components stem components 120A, 120B. Component 320 may also include a transverse hole 336 that extends through the entirety of component 320 and is sized and configured to receive a tool for disengaging a tapered connection. Although hole 336 is shown as extending through the entirety of component 320, it should be understood that hole 336 may extend only partially through component 320.

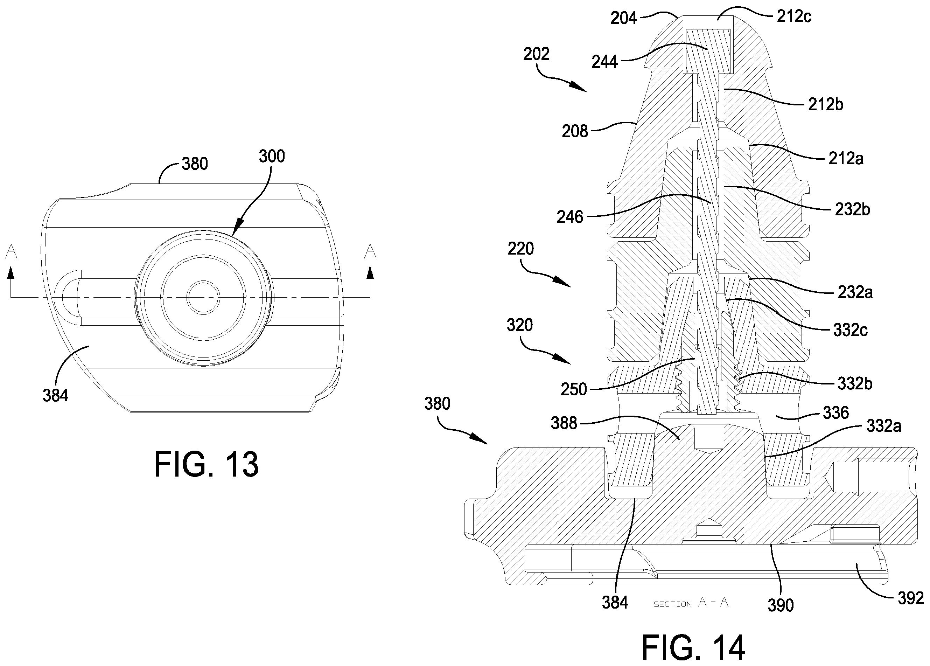

As best seen in FIGS. 10 and 14, tibia tray component 380 may have a body 382 with an upper surface 384 from which a protrusion 386 extends. In some embodiments, protrusion 386 may be provided without an opening that extends entirely through the protrusion 386. Protrusion 386 may be tapered along its length and sized such that protrusion 386 may be configured to be at least partially received in the first portion 332a of the opening 332 defined by the component 320. Tibia tray component may include an inner surface 390 between a pair of spaced apart rails 392, which may be sized and configured to receive an artificial articular surface.

In use, the patient's anatomy may be prepared, such as by reaming an intramedullary canal in bone. The tensioning wire 242 may be preloaded into stem component 202. If tensioning wire 242 is not preloaded into stem component 202, then the flexible shaft portion 246 may be inserted into the opening 212 such that the flexible shaft portion 246 is received first in the third portion 212c, then in the second portion 212b and the first portion 212c. With the tensioning wire 242 coupled to the first component 202, the first component 202 may be inserted into the intramedullary canal. In some embodiments, the first component 202 may be inserted into the intramedullary canal by inserting the first component 202 into a resected joint space formed between a tibia and a talus such that the first component enters the joint space in an anterior-to-posterior direction. The first component 202 may be engaged by a tool and pressed into the intramedullary canal.

A second component, such as component 220, may be coupled to the first component 202 in situ by inserting the component 220 into the resected joint space in an anterior-to-posterior direction. The flexible shaft portion 246 that extends from the first component 202 may be inserted into the opening 232 defined by the component 220. The component 220 may be pressed into engagement with the first component 202 by aligning the two components and inserting the tapered region 228 of the component 220 into the first portion 212a of the opening defined by the first component 202. The construct of the first component 202 and the second component 220 may be advanced farther into the intramedullary canal.

A third component, such as component 320, may be coupled to the construct of the first component 202 and second component 220 in situ. For example, the third component 320 may be inserted into the resected joint space in an anterior-to-posterior direction. The flexible shaft portion 246 that extends from the second end 226 of the component 220 may be inserted into the opening 332 defined by the component 320 such that at least a portion of the flexible shaft portion 246 extends from the second end 326 of component 320. If collet 250 is coupled to component 320 when the component 320 is inserted into the resected joint space, then the flexible shaft portion 246 may be received in the hole 266 defined by the collet 250. If collet 250 is not coupled to component 320 when the component 320 is inserted into the resected joint space, then the collet 250 may be coupled to component 320 in situ by inserting the flexible shaft portion 246 into the hole 266 defined by the collet 250 and inserting collet 250 into the opening 332 defined by component 320.

The stem may be compressed and locked together by using coupling member 240. For example, tension may be applied to tensioning wire 242 using a pair of pliers, vice grips, or other suitable tool. Pulling on the flexible shaft portion 246 while applying pressure to the second end 326 of stem component 320 compresses the stem components together. The collet 250 may be rotated such that the threads 264 of collet 250 engage the threaded portion 332a of the opening defined by component 320. The advancement of the collet 250 into the opening 332 defined by component 320 results in the flexible arms 260 of the collet 250 being compressed and clamping onto the flexible shaft portion 246 to lock the stem components together. Any excess portion of the flexible shaft portion 246 may be cut using a wire cutter, snips, or any other suitable tool.

The tibia tray component 380 may be coupled to the stem 300. For example, the tibia tray component 380 may be inserted into the resected joint space such that the protrusion 386 may be aligned with the opening 232 defined by the component 320 of the stem 300. The protrusion 386 of the tibia tray component 380 is pressed into the first portion 332a to form a tapered connection. An artificial articular surface may be coupled to the tibia tray component 380 as will be understood by one of ordinary skill in the art.

The tibia stem component and/or the tibia tray component in the various embodiments of the prosthesis disclosed herein can be made of any total joint material of materials commonly used in the prosthetic art, including, but not limited to, metals, ceramics, titanium, titanium alloys, tantalum, chrome cobalt, surgical steel, polyethylene, absorbable polymer, or any other total joint replacement metal and/or ceramic. In some embodiments, the tibia stem component and/or the tibia tray component can comprise a coating of 3D printed Biofoam^{™}, Adaptis^{™}, porous metal, sintered glass, artificial bone, any uncemented metal or ceramic surface, or a combination thereof that would promote bony in-growth. As noted above, other surface texturing, including grit blasting, may be applied to the outer surfaces of the stem and/or tibia tray component to facilitate bony ingrowth/ongrowth. The tibia stem component and/or the tibia tray component can further be covered with one or more coatings such as antimicrobial, antithrombotic, and osteoinductive agents, or a combination thereof. In embodiments where the above-mentioned porous coating is provided, these agents can further be carried in a biodegradable carrier material with which the pores in the porous coating can be impregnated.

Although the examples of the inventive structures illustrated herein are exemplified as tibia stem and tray components of an ankle replacement prosthesis, the inventive structures are equally applicable in other physical joints in human or animal skeleton.

It will be understood that the foregoing description is of exemplary embodiments of this invention, and that the invention is not limited to the specific forms shown. Modifications may be made in the design and arrangement of the elements without departing from the scope of the invention, which is defined by the claims.

## Claims

1. A tibia stem prosthesis (100) for an ankle replacement, comprising:
a first component (102) having a first body extending from a first end (104) to a second end (106), the second end (106) of the first component (102) defining a first opening (112) that extends inwardly toward the first end (104) of the first component (102); and a second component (120) having a second body (122) extending from a first end (124) to a second end (126); **characterised in that** the first opening (112) of the first component (102) includes a first portion (112a) having a first diameter and a second portion (112b) having a second diameter that is different from the first diameter;
wherein the second body (122) defines a second opening (132) that extends through the second body (122) from the first end (124) of the second body (122) to the second end (126) of the second body (122); and wherein the tibia stem prosthesis further comprises
a coupling member (140) configured to couple the first component (102) and the second component (120) by compressing the first component (102) and the second component (120), the coupling member (140) including a shaft portion (146) and a head portion (144), the shaft portion (146) sized and configured to be received in the first opening (112) and the second opening (132).

2. The tibia stem prosthesis (100) of claim 1, wherein the second body (122) of the second component (120) includes a protrusion extending from a shoulder, the protrusion sized and configured to be at least partially received in the first portion of the first opening (112) defined by the first component (102).

3. The tibia stem prosthesis (100) of claim 2, wherein: each of the first portion of the first opening (112) and the protrusion of the second body (122) includes a respective taper, and
the respective tapers are configured to engage one another.

4. The tibia stem prosthesis (100) of claim 3, wherein
the second portion of the first opening (112) includes at least one first thread,
the shaft of the coupling member (140) includes at least one second thread, and
the at least one second thread is configured to engage the at least one first thread, wherein the second opening (132) defined by the second component (120) optionally includes a first portion, a second portion, and a third portion, the first portion including a taper, the second portion sized and configured to at least partially receive the head of the coupling member (140), and the third portion has a diameter that is greater than a diameter of the shaft of the coupling member (140) and smaller than a diameter of the head portion (144) of the coupling member (140).

5. The tibia stem prosthesis (100) of any of claims 3-4, wherein:
the first opening (112) includes a third portion,
the second portion of the first opening (112) is located between the first portion of the first opening (112) and the third portion of the first opening (112), and
the first opening (112) is sized and configured to receive the head portion (144) of the coupling member (140).

6. The tibia stem prosthesis (100) of claim 5, wherein the coupling member (140) includes
a tensioning wire comprising the head portion (144) and the shaft portion (146), and
a collet defining a third opening and including a plurality of flexible arms, the third opening sized and configured to receive the shaft portion (146) of the tensioning wire.

7. The tibia stem prosthesis (100) of claim 6, wherein the second opening (132) defined by the second component (120) includes a first portion and a second portion.

8. The tibia stem prosthesis (100) of claim 7, wherein
the first portion of the second opening (132) includes at least one thread sized and configured to be engaged by a thread disposed on an outer surface of the collet, and
the second portion includes taper configured to engage the plurality of flexible arms; or wherein:
the first portion of the second opening (132) includes a taper sized and configured to engage a protrusion of another component.

9. The tibia stem prosthesis (100) of claim 7, wherein:
the first portion of the second opening (132) includes a taper sized and configured to engage a protrusion of another component,
and wherein the prosthesis (100) is further comprising a third component having a third body, the third body including:
a pair of spaced apart rails, and
a protrusion extending from an upper surface of the third body, the protrusion sized and configured to be received in at least one of the first opening (112) or the second opening (132).

10. The tibia stem prosthesis (100) of claim 9, wherein the third body defines a fourth opening sized and configured to receive the collet therein, wherein the fourth opening defined by the third body optionally includes a first portion and a second portion, the first portion including at least one thread configured to engage at least one thread disposed on an outer surface of the collet, and the second portion including a taper configured to engage the plurality of flexible arms.

11. The tibia stem prosthesis (100) of any of the preceding claims, wherein the first portion includes at least one anti-rotation feature extending from an outer surface of the first body, wherein optionally the at least one anti-rotation feature includes a plurality of fins extending from the first end (104) of the first body toward the second end (106) of the first body.

12. The tibia stem prosthesis (100) of any of the preceding claims, wherein the first portion includes surface texturing to promote bone ingrowth/ongrowth.

13. The tibia stem prosthesis (100) of claim 12, wherein the surface texturing includes at least one groove formed in an outer surface of the first body, wherein the at least one groove optionally includes at least one circumferential groove, and/or at least one helical groove

14. The tibia stem prosthesis (100) of any of claims 12-13, wherein the surface texturing includes a texture formed by grit blasting.

15. The tibia stem prosthesis (100) of any of the preceding claims, wherein the first body includes an elongate cylindrical section disposed between the first end (104) of the first body and the second end (106) of the first body.

## Patentansprüche

1. Tibiaschaftprothese (100) für einen Sprunggelenkersatz, umfassend:
eine erste Komponente (102), die einen ersten Körper aufweist, der sich von einem ersten Ende (104) zu einem zweiten Ende (106) erstreckt, wobei das zweite Ende (106) der ersten Komponente (102) eine erste Öffnung (112) definiert, die sich nach innen in Richtung des ersten Endes (104) der ersten Komponente (102) erstreckt; und eine zweite Komponente (120), die einen zweiten Körper (122) aufweist, der sich von einem ersten Ende (124) zu einem zweiten Ende (126) erstreckt; **dadurch gekennzeichnet, dass** die erste Öffnung (112) der ersten Komponente (102) einen ersten Abschnitt (112a) mit einem ersten Durchmesser und einen zweiten Abschnitt (112b) mit einem zweiten Durchmesser, der sich von dem ersten Durchmesser unterscheidet, umfasst; wobei der zweite Körper (122) eine zweite Öffnung (132) definiert, die sich durch den zweiten Körper (122) von dem ersten Ende (124) des zweiten Körpers (122) zu dem zweiten Ende (126) des zweiten Körpers (122) erstreckt; und wobei die Tibiaschaftprothese ferner umfasst:
ein Kopplungselement (140), das zum Koppeln der ersten Komponente (102) und der zweiten Komponente (120) durch Komprimieren der ersten Komponente (102) und der zweiten Komponente (120) konfiguriert ist, wobei das Kopplungselement (140) einen Schaftabschnitt (146) und einen Kopfabschnitt (144) umfasst, wobei der Schaftabschnitt (146) so bemessen und konfiguriert ist, um in der ersten Öffnung (112) und der zweiten Öffnung (132) aufgenommen zu werden.

2. Tibiaschaftprothese (100) nach Anspruch 1, wobei der zweite Körper (122) der zweiten Komponente (120) einen sich von einer Schulter erstreckenden Vorsprung umfasst, wobei der Vorsprung so bemessen und konfiguriert ist, um mindestens teilweise in dem durch die erste Komponente (102) definierten ersten Abschnitt der ersten Öffnung (112) aufgenommen zu werden.

3. Tibiaschaftprothese (100) nach Anspruch 2, wobei:
jeder von dem ersten Abschnitt der ersten Öffnung (112) und dem Vorsprung des zweiten Körpers (122) eine jeweilige Verjüngung umfasst, und
die jeweiligen Verjüngungen konfiguriert sind, um miteinander in Eingriff zu kommen.

4. Tibiaschaftprothese (100) nach Anspruch 3, wobei:
der zweite Abschnitt der ersten Öffnung (112) mindestens ein erstes Gewinde umfasst,
der Schaft des Kopplungselements (140) mindestens ein zweites Gewinde umfasst, und
das mindestens eine zweite Gewinde zum Eingreifen in das mindestens eine erste Gewinde konfiguriert ist, wobei die durch die zweite Komponente (120) definierte zweite Öffnung (132) optional einen ersten Abschnitt, einen zweiten Abschnitt und einen dritten Abschnitt umfasst, wobei der erste Abschnitt eine Verjüngung umfasst, der zweite Abschnitt so bemessen und konfiguriert ist, um den Kopf des Kopplungselements (140) mindestens teilweise aufzunehmen, und der dritte Abschnitt einen Durchmesser aufweist, der größer als ein Durchmesser des Schafts des Kopplungselements (140) und kleiner als ein Durchmesser des Kopfabschnitts (144) des Kopplungselements (140) ist.

5. Tibiaschaftprothese (100) nach einem der Ansprüche 3 bis 4, wobei:
die erste Öffnung (112) einen dritten Abschnitt umfasst,
der zweite Abschnitt der ersten Öffnung (112) zwischen dem ersten Abschnitt der ersten Öffnung (112) und dem dritten Abschnitt der ersten Öffnung (112) angeordnet ist, und
die erste Öffnung (112) so bemessen und konfiguriert ist, um den Kopfabschnitt (144) des Kopplungselements (140) aufzunehmen.

6. Tibiaschaftprothese (100) nach Anspruch 5, wobei das Kopplungselement (140) umfasst:
einen Spanndraht, der den Kopfabschnitt (144) und den Schaftabschnitt (146) umfasst, und
eine Spannhülse, die eine dritte Öffnung definiert und eine Vielzahl von flexiblen Armen umfasst, wobei die dritte Öffnung so bemessen und konfiguriert ist, um den Schaftabschnitt (146) des Spanndrahts aufzunehmen.

7. Tibiaschaftprothese (100) nach Anspruch 6, wobei die durch die zweite Komponente (120) definierte zweite Öffnung (132) einen ersten Abschnitt und einen zweiten Abschnitt umfasst.

8. Tibiaschaftprothese (100) nach Anspruch 7, wobei:
der erste Abschnitt der zweiten Öffnung (132) mindestens ein Gewinde umfasst, das so bemessen und konfiguriert ist, um mit einem an einer Außenoberfläche der Spannhülse angeordneten Gewinde in Eingriff zu kommen, und
der zweite Abschnitt eine Verjüngung umfasst, die konfiguriert ist, um mit der Vielzahl von flexiblen Armen in Eingriff zu kommen; oder wobei:
der erste Abschnitt der zweiten Öffnung (132) eine Verjüngung umfasst, die so bemessen und konfiguriert ist, um mit einem Vorsprung einer anderen Komponente in Eingriff zu kommen.

9. Tibiaschaftprothese (100) nach Anspruch 7, wobei:
der erste Abschnitt der zweiten Öffnung (132) eine Verjüngung umfasst, die so bemessen und konfiguriert ist, um mit einem Vorsprung einer anderen Komponente in Eingriff zu kommen,
und wobei die Prothese (100) ferner eine dritte Komponente umfasst, die einen dritten Körper aufweist, wobei der dritte Körper Folgendes umfasst:
ein Paar beabstandeter Schienen, und
einen Vorsprung, der sich von einer oberen Oberfläche des dritten Körpers erstreckt, wobei der Vorsprung so bemessen und konfiguriert ist, um in mindestens einer von der ersten Öffnung (112) oder der zweiten Öffnung (132) aufgenommen zu werden.

10. Tibiaschaftprothese (100) nach Anspruch 9, wobei der dritte Körper eine vierte Öffnung definiert, die so bemessen und konfiguriert ist, um die Spannhülse darin aufzunehmen, wobei die durch den dritten Körper definierte vierte Öffnung optional einen ersten Abschnitt und einen zweiten Abschnitt umfasst, wobei der erste Abschnitt mindestens ein Gewinde umfasst, das zum Eingreifen in mindestens ein an einer Außenoberfläche der Spannhülse angeordnetes Gewinde konfiguriert ist, und der zweite Abschnitt eine Verjüngung umfasst, die konfiguriert ist, um mit der Vielzahl von flexiblen Armen in Eingriff zu kommen.

11. Tibiaschaftprothese (100) nach einem der vorstehenden Ansprüche, wobei der erste Abschnitt mindestens ein Antirotationsmerkmal umfasst, das sich von einer Außenoberfläche des ersten Körpers erstreckt, wobei optional das mindestens eine Antirotationsmerkmal eine Vielzahl von Rippen umfasst, die sich von dem ersten Ende (104) des ersten Körpers in Richtung des zweiten Endes (106) des ersten Körpers erstrecken.

12. Tibiaschaftprothese (100) nach einem der vorstehenden Ansprüche, wobei der erste Abschnitt eine Oberflächentexturierung umfasst, um das Einwachsen/Anwachsen von Knochen zu fördern.

13. Tibiaschaftprothese (100) nach Anspruch 12, wobei die Oberflächentexturierung mindestens eine Nut umfasst, die in einer Außenoberfläche des ersten Körpers gebildet ist, wobei die mindestens eine Nut optional mindestens eine umlaufende Nut und/oder mindestens eine schraubenförmige Nut umfasst.

14. Tibiaschaftprothese (100) nach einem der Ansprüche 12 bis 13, wobei die Oberflächentexturierung eine durch Sandstrahlen gebildete Textur umfasst.

15. Tibiaschaftprothese (100) nach einem der vorstehenden Ansprüche, wobei der erste Körper einen länglichen zylindrischen Abschnitt umfasst, der zwischen dem ersten Ende (104) des ersten Körpers und dem zweiten Ende (106) des ersten Körpers angeordnet ist.

## Revendications

1. Prothèse à tige tibiale (100) destinée au remplacement de la cheville, comprenant :
un premier composant (102) ayant un premier corps s'étendant à partir d'une première extrémité (104) jusqu'à une deuxième extrémité (106), la deuxième extrémité (106) du premier composant (102) définissant une première ouverture (112) qui s'étend vers l'intérieur vers la première extrémité (104) du premier composant (102) ; et un deuxième composant (120) ayant un deuxième corps (122) s'étendant à partir d'une première extrémité (124) jusqu'à une deuxième extrémité (126) ; **caractérisée en ce que** la première ouverture (112) du premier composant (102) comprend une première partie (112a) ayant un premier diamètre et une deuxième partie (112b) ayant un deuxième diamètre qui est différent du premier diamètre ;
dans laquelle le deuxième corps (122) définit une deuxième ouverture (132) qui s'étend à travers le deuxième corps (122) à partir de la première extrémité (124) du deuxième corps (122) jusqu'à la deuxième extrémité (126) du deuxième corps (122) ; et
dans laquelle la prothèse à tige tibiale comprend en outre :
un élément de couplage (140) configuré pour coupler le premier composant (102) et le deuxième composant (120) en comprimant le premier composant (102) et le deuxième composant (120), l'élément de couplage (140) comprenant une partie d'arbre (146) et une partie de tête (144), la partie d'arbre (146) étant dimensionnée et configurée pour être reçue dans la première ouverture (112) et la deuxième ouverture (132).

2. Prothèse à tige tibiale (100) selon la revendication 1, dans laquelle le deuxième corps (122) du deuxième composant (120) comprend une saillie s'étendant à partir d'un épaulement, la saillie étant dimensionnée et configurée pour être au moins partiellement reçue dans la première partie de la première ouverture (112) définie par le premier composant (102).

3. Prothèse à tige tibiale (100) selon la revendication 2, dans laquelle :
chacune parmi la première partie de la première ouverture (112) et la saillie du deuxième corps (122) comprend une partie conique respective, et
les parties coniques respectives sont configurées pour se mettre en prise l'une avec l'autre.

4. Prothèse à tige tibiale (100) selon la revendication 3, dans laquelle :
la deuxième partie de la première ouverture (112) comprend au moins un premier filetage,
l'arbre de l'élément de couplage (140) comprend au moins un deuxième filetage, et
le au moins un deuxième filetage est configuré pour mettre en prise le au moins un premier filetage, dans laquelle la deuxième ouverture (132) définie par le deuxième composant (120) comprend facultativement une première partie, une deuxième partie et une troisième partie, la première partie comprenant une partie conique, la deuxième partie étant dimensionnée et configurée pour recevoir, au moins partiellement, la tête de l'élément de couplage (140), et la troisième partie a un diamètre qui est supérieur à un diamètre de l'arbre de l'élément de couplage (140) et inférieur à un diamètre de la partie de tête (144) de l'élément de couplage (140).

5. Prothèse à tige tibiale (100) selon l'une quelconque des revendications 3 à 4, dans laquelle :
la première ouverture (112) comprend une troisième partie,
la deuxième partie de la première ouverture (112) est située entre la première partie de la première ouverture (112) et la troisième partie de la première ouverture (112), et
la première ouverture (112) est dimensionnée et configurée pour recevoir la partie de tête (144) de l'élément de couplage (140).

6. Prothèse à tige tibiale (100) selon la revendication 5, dans laquelle l'élément de couplage (140) comprend :
un fil de tension comprenant la partie de tête (144) et la partie d'arbre (146), et
un collet définissant une troisième ouverture et comprenant une pluralité de bras flexibles, la troisième ouverture étant dimensionnée et configurée pour recevoir la partie d'arbre (146) du fil de tension.

7. Prothèse à tige tibiale (100) selon la revendication 6, dans laquelle la deuxième ouverture (132) définie par le deuxième composant (120) comprend une première partie et une deuxième partie.

8. Prothèse à tige tibiale (100) selon la revendication 7, dans laquelle :
la première partie de la deuxième ouverture (132) comprend au moins un filetage dimensionné et configuré pour être mis en prise par un filetage disposé sur une surface externe du collet, et
la deuxième partie comprend une partie conique configurée pour mettre en prise la pluralité de bras flexibles ; ou dans laquelle :
la première partie de la deuxième ouverture (132) comprend une partie conique dimensionnée et configurée pour mettre en prise une saillie d'un autre composant.

9. Prothèse à tige tibiale (100) selon la revendication 7, dans laquelle :
la première partie de la deuxième ouverture (132) comprend une partie conique dimensionnée et configurée pour mettre en prise une saillie d'un autre composant,
et dans laquelle la prothèse (100) comprend en outre un troisième composant ayant un troisième corps, le troisième corps comprenant :
une paire de rails espacés, et
une saillie s'étendant à partir d'une surface supérieure du troisième corps, la saillie étant dimensionnée et configurée pour être reçue dans au moins l'une parmi la première ouverture (112) ou la deuxième ouverture (132).

10. Prothèse à tige tibiale (100) selon la revendication 9, dans laquelle le troisième corps définit une quatrième ouverture dimensionnée et configurée pour recevoir le collet dans cette dernière, dans laquelle la quatrième ouverture définie par le troisième corps comprend facultativement une première partie et une deuxième partie, la première partie comprenant au moins un filetage configuré pour mettre en prise au moins un filetage disposé sur une surface externe du collet, et la deuxième partie comprenant une partie conique configurée pour mettre en prise la pluralité des bras flexibles.

11. Prothèse à tige tibiale (100) selon l'une quelconque des revendications précédentes, dans laquelle la première partie comprend au moins une caractéristique anti-rotation s'étendant à partir d'une surface externe du premier corps, dans laquelle facultativement la au moins une caractéristique anti-rotation comprend une pluralité d'ailettes s'étendant à partir de la première extrémité (104) du premier corps vers la deuxième extrémité (106) du premier corps.

12. Prothèse à tige tibiale (100) selon l'une quelconque des revendications précédentes, dans laquelle la première partie comprend une texturation de surface destinée à favoriser la croissance osseuse dans et sur la surface.

13. Prothèse à tige tibiale (100) selon la revendication 12, dans laquelle la texturation de surface comprend au moins une rainure formée dans une surface externe du premier corps, dans laquelle la au moins une rainure comprend facultativement au moins une rainure circonférentielle, et/ou au moins une rainure hélicoïdale.

14. Prothèse à tige tibiale (100) selon l'une quelconque des revendications 12 à 13, dans laquelle la texturation de surface comprend une texture formée par grenaillage.

15. Prothèse à tige tibiale (100) selon l'une quelconque des revendications précédentes, dans laquelle le premier corps comprend une section cylindrique allongée disposée entre la première extrémité (104) du premier corps et la deuxième extrémité (106) du premier corps.
